# EUROPEAN PATENT SPECIFICATION

(11) **EP 4 642 512 B1**
(45) Date of publication and mention of the grant of the patent: **18.11.2026**
(21) Application number: 23837205.6
(22) Date of filing: 20.12.2023
(51) Int. Cl.: A61N 1/04, A61N 1/39

(54) **ELECTRODE PAD ASSEMBLY FOR MULTIPLE DEFIBRILLATION VECTOR THERAPY**
ELEKTRODENPOLSTER FÜR THERAPIE MIT MEHREREN DEFIBRILLATIONSVEKTOREN
ASSEMBLAGE D'ÉLECTRODES POUR THÉRAPIE PAR VECTEURS DE DÉFIBRILLATION MULTIPLES

(30) Priority: 31.12.2022 US 202263436507 P
(43) Date of publication of application: 05.11.2025
(73) Proprietor: Everest Acquisition Entity, LLC, Wilmington, DE 19805 (US)
(72) Inventor: LIU, Chenguang, 5656 AG Eindhoven (NL); JORGENSON, Dawn, Blilie, 5656 AG Eindhoven (NL)
(74) Representative: Modiano, Gabriella Diana
(86) International application number: PCT/EP2023/086787
(87) International publication number: WO 2024/141354

(56) References cited:
- US-A1- 2006 155 336
- US-A1- 2006 155 354
- US-A1- 2007 100 381
- US-A1- 2010 114 218
- US-A1- 2019 344 068
- US-A1- 2022 280 377
- US-B2- 9 498 152

## Description

### FIELD OF THE INVENTION

The present disclosure generally relates to defibrillation for a patient experiencing cardiac arrythmias, and more particularly to an electrode pad assembly facilitating multiple defibrillation vector therapies (e.g., vector-change defibrillation, double sequential defibrillation and double simultaneous defibrillation).

### BACKGROUND OF THE INVENTION

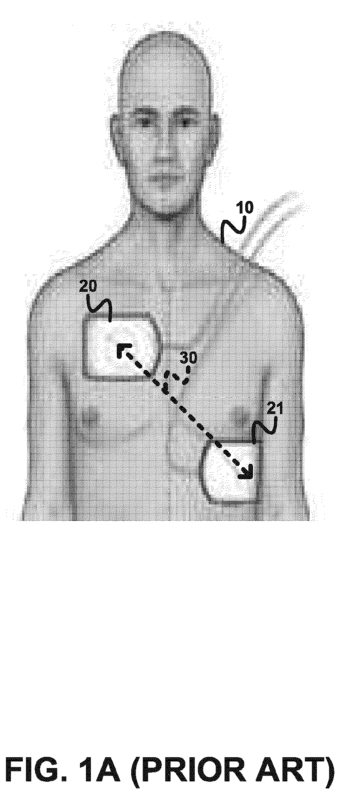

FIG. 1A illustrates a standard anterolateral electrode placement on an adult male patient 10 as known in the art of the present disclosure. Specifically, an electrode pad 20 is placed on an upper right torso of adult male patient 10 below a collarbone of patient 10, and an electrode pad 21 is placed on a lower left torso of adult male patient 10 below a pectoral muscle of adult male patient 10. During defibrillation of patient 10, an anterolateral defibrillation vector 30 in terms of defibrillation waveform magnitude and direction extends between electrode pads 20 and 21 to thereby deliver a shock to a heart of adult male patient 10.

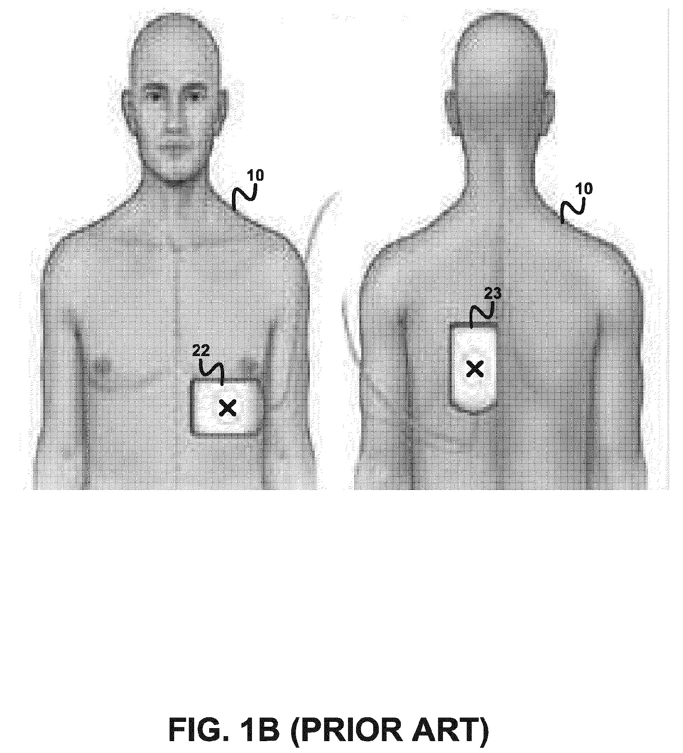

FIG. 1B illustrates an anteroposterior electrode placement on adult male patient 10 as known in the art of the present disclosure. Specifically, an electrode pad 22 is placed over a cardiac apex of patient 10 between the midline of the chest and nipple on patient 10, and an electrode pad 23 is placed to a left of a spine of patient 10 below a scapula of patient 10 at the heart level. During defibrillation of patient 10, an anteroposterior defibrillation vector (symbolized by the X in electrode pads 22 and 23) in terms of a defibrillation waveform magnitude and direction extends between electrode pads 22 and 23 to thereby deliver a shock to a heart of adult male patient 10.

During therapy of adult male patient 10, a switch between the standard anterolateral electrode placement of FIG. 1A to the anteroposterior electrode placement of FIG. 1B is known in the art of the present disclosure as a vector-change defibrillation.

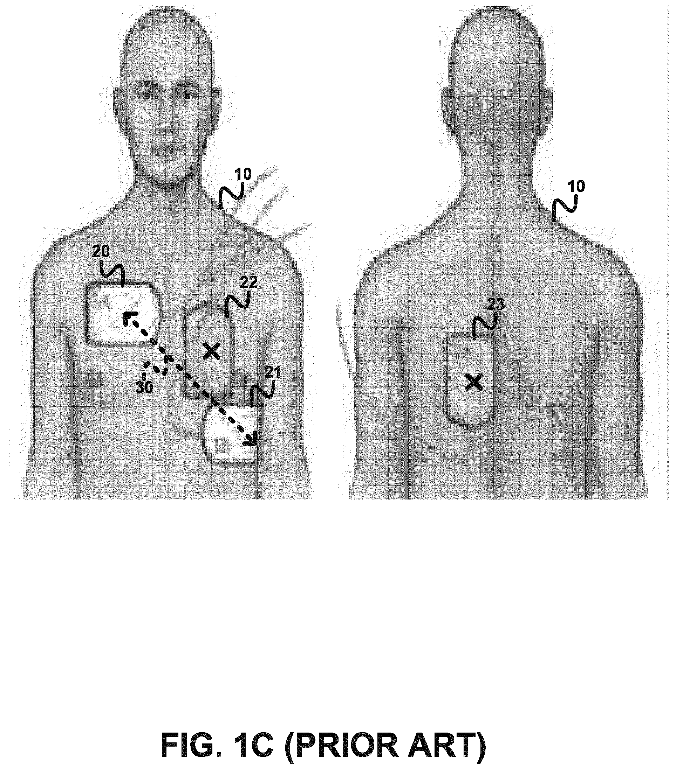

FIG. 1C illustrates a double sequential electrode placement on adult male patient 10 as known in the art of the present disclosure. Specifically, as shown in FIG. 1C, electrode pad 20 is placed on an upper right torso of adult male patient 10 below a collarbone of patient 10, and electrode pad 21 is placed on a lower left torso of adult male patient 10 below a pectoral muscle of adult male patient 10. Further, electrode pad 22 is placed on an anterior wall of a left chest muscle of adult male patient 10, and electrode pad 23 is placed to a left of a spine of patient 10 below a scapula of patient 10 at the heart level.

During defibrillation of patient 10, in a sequential order, anterolateral defibrillation vector 30 in terms of defibrillation waveform magnitude and direction extends between electrode pads 20 and 21 to thereby deliver a first shock or a second shock to a heart of adult male patient 10, and anteroposterior defibrillation vector (symbolized by the X in electrode pads 22 and 23) in terms of defibrillation waveform magnitude and direction extends between electrode pads 22 and 23 to thereby deliver the first shock or a second shock to a heart of adult male patient 10.

This is known in the art of the present disclosure as a double sequential defibrillation.

Alternatively during defibrillation of patient 10, anterolateral defibrillation vector 30 in terms of defibrillation waveform magnitude and direction extends between electrode pads 20 and 21 to thereby deliver a shock a heart of adult male patient 10, and anteroposterior defibrillation vector (symbolized by the X in electrode pads 22 and 23) in terms of defibrillation waveform magnitude and direction extends between electrode pads 22 and 23 to thereby deliver a simultaneous shock to a heart of adult male patient 10.

This is known in the art of the present disclosure as a double simultaneous defibrillation.

US 2010/0114218 A1, US 2022/0280377 A1, US 2006/0155354 A1 and US 2006/0155336 A1 disclose prior art examples of multiple defibrillation vector therapies.

The field of resuscitation is heavily focused on increasing a quality of care set forth in the prior art by identifying and providing optimal CPR/shock treatment for a patient experiencing cardiac arrythmia.

### SUMMARY OF THE INVENTION

The present disclosure is directed to a defibrillation electrode pad therapy that facilitates multiple defibrillation vector therapy including a vector-change defibrillation (excluding any movement of electrodes on a patient), a double sequential defibrillation (particularly, from a single defibrillator) and/or a double simultaneous defibrillation (particularly, from a single defibrillator).

The present disclosure may be embodied as (1) a defibrillation electrode pad assembly for a multiple defibrillation vector therapy, and (2) a multiple defibrillation vector therapy system. The invention is defined by the independent claims. The dependent claims define advantageous embodiments.

Various exemplary embodiments of a defibrillation electrode pad assembly of the present disclosure encompass incorporating an integration of a first electrode pad and a second electrode pad with an anterior-anterior band, and an integration of a third electrode pad and a fourth electrode pad with an anterior-posterior band. During the multiple defibrillation vector therapy of the patient, the first electrode pad and the third electrode pad are operable to generate an anterior-anterior defibrillation vector when the anterior-anterior band and the anterior-posterior band are utilized to place the first electrode pad and the third electrode pad on the anterior of the patient, and the second electrode pad and the fourth electrode pad are operable to generate an anterior-posterior defibrillation vector when the anterior-anterior band and the anterior-posterior band are utilized to place the second electrode pad on the anterior of the patient and the fourth electrode pad on the posterior of the patient.

Various exemplary embodiments of a multiple defibrillation vector therapy system of the present disclosure encompass a defibrillation electrode pad assembly of the present disclosure and a defibrillator for controlling a generation of an anterior-anterior defibrillation vector between the first electrode pad and the third electrode pad when the anterior-anterior band and the anterior-posterior band are utilized to place the first electrode pad and the third electrode pad on the anterior of the patient, and controlling a generation of an anterior-posterior defibrillation vector between the second electrode pad and the fourth electrode pad when the anterior-anterior band and the anterior-posterior band are utilized to place the second electrode pad on the anterior of the patient and the fourth electrode pad on the posterior of the patient.

The foregoing exemplary embodiments and other embodiments of the present disclosure as well as various structures and advantages of the present disclosure will become further apparent to those having ordinary skill in the art from the following detailed description of various embodiments of the present disclosure read in conjunction with the accompanying drawings. The detailed description and drawings are merely illustrative of the present disclosure rather than limiting, the scope of the present disclosure being defined by the appended claims and equivalents thereof.

### BRIEF DESCRIPTION OF THE DRAWINGS

The present disclosure will present in detail the following description of exemplary embodiments with reference to the following figures wherein:
FIGS. 1A-1C illustrate conventional electrode pad placements for defibrillation of a patient as known in the art of the present disclosure;
FIG. 2A illustrates exemplary embodiments of a band in accordance with the present disclosure;
FIG. 2B illustrates an exemplary embodiment of electrode pads as known in the art of the present disclosure;
FIG. 3A illustrates a first exemplary embodiment of a defibrillation electrode pad assembly in accordance with the present disclosure;
FIG. 3B illustrates a second exemplary embodiment of a defibrillation electrode pad assembly in accordance with the present disclosure;
FIG. 4A illustrates a first exemplary embodiment of a multiple defibrillation vector system in accordance with the present disclosure;
FIG. 4B illustrates a second exemplary embodiment of a multiple defibrillation vector system in accordance with the present disclosure;
FIG. 5 illustrates a flowchart representative of an exemplary use of a vector-change defibrillation system in accordance with the present disclosure;
FIG. 6 illustrates a flowchart representative of a first exemplary use of a double sequential defibrillation system in accordance with the present disclosure;
FIG. 7 illustrates a flowchart representative of a second exemplary use of a double sequential defibrillation system in accordance with the present disclosure; and
FIG. 8 illustrates a flowchart representative of an exemplary use of double simultaneous defibrillation system in accordance with the present disclosure.

### DETAILED DESCRIPTION OF THE EXEMPLARY EMBODIMENTS

The present disclosure is directed to a defibrillation electrode pad therapy that facilitates multiple defibrillation vector therapy including a vector-change defibrillation (excluding any movement of electrodes on a patient), a double sequential defibrillation (particularly, from a single defibrillator) and/or a double simultaneous defibrillation (particularly, from a single defibrillator).

In practice, a defibrillation electrode pad assembly of the present disclosure employs an incorporates an integration of a first electrode pad and a second electrode pad with an anterior-anterior band, and an integration of a third electrode pad and a fourth electrode pad with an anterior-posterior band. The defibrillation electrode pad assembly may also employ additional electrode pads and/or additional bands.

For purpose of claiming and describing the present disclosure, the term "band" broadly encompasses any material, as known in the art of the present disclosure or hereinafter conceived, that may be applied to and/or extend over a patient for facilitating a defibrillation of the patient via electrode pads. Examples of a band include, but are not limited to, an elastic bandage and elastic clothing material as known in the art of the present disclosure. In practice, the band may have any geometrical configuration in support of a placement of one or more electrode pads on a patient to facilitate a defibrillation of the patient.

More particularly, any band of the present disclosure designated as anterior-anterior band will have a geometric configuration and material composition suitable for an application to and/or extension over anterior of a patient.

Additionally, any band of the present disclosure designated as anterior-posterior band will have a geometric configuration and material composition suitable for an application to an anterior and/or a posterior of the patient and/or extension over the anterior and a posterior of the patient.

For purpose of claiming and describing the present disclosure, the term "electrode pad" broadly encompasses any device, as known in the art of the of the present disclosure or hereinafter conceived, including an electrode for conducting shock energy from a defibrillator to a patient and material for adhering the electrode to the patient. Examples of an electrode pad include, but are not limited to, electrode pads utilized in the Automated External Defibrillators (AED) as known in the art of the present disclosure. In practice, the electrode pad may have any geometrical configuration in support of a placement of the electric pad on a patient of a defibrillation of the patient.

For purpose of claiming and describing the present disclosure, the term "integration" broadly encompasses permanent or impermanent type of combination, merger, adjoining, mounting or otherwise that constitutes a band and one or more electrode pads as a single item.

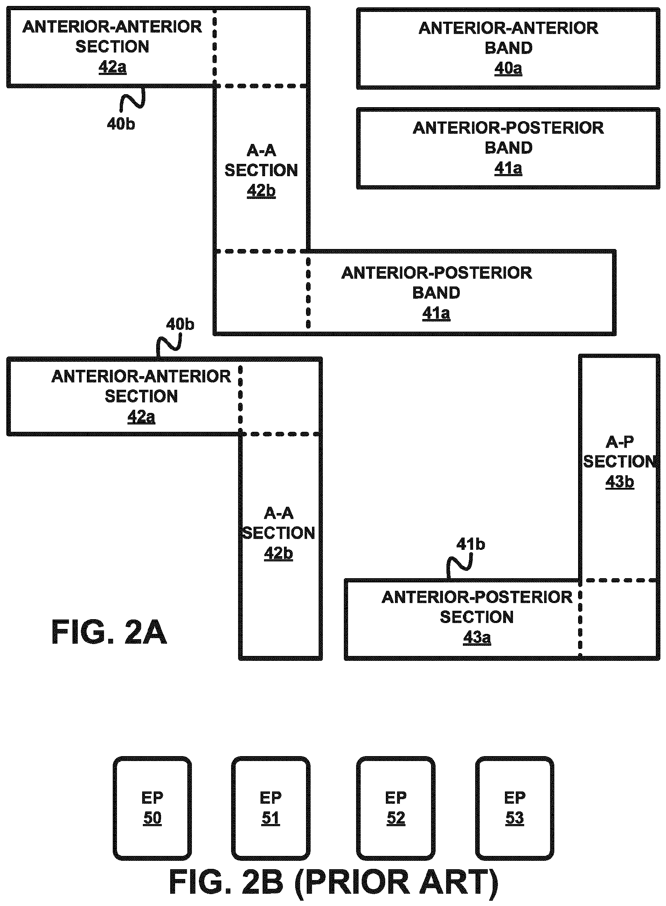

To facilitate an understanding of the present disclosure, the following description of FIGS. 2A-3B teaches exemplary embodiments of a defibrillation electrode pad assembly in accordance with the present disclosure. From the description of FIGS. 2A-3B, those having ordinary skill in the art of the present disclosure will appreciate how to apply the present disclosure to make and use additional embodiments of defibrillation electrode pad assembly in accordance with the present disclosure.

Referring to FIG. 2A, several exemplary embodiments of bands for the present disclosure are shown.

In a first exemplary embodiment, FIG. 2A illustrates an anterior-anterior band 40a having a prismatic configuration designed to be applied to and/or extended over an anterior of a patient, and an anterior-posterior band 41a, having a prismatic configuration designed to be applied to and/or extended over an anterior and a posterior of a patient. An anterior-anterior band 40a and anterior-posterior band 41a are segregated as separate bands.

In a second exemplary embodiment, FIG. 2A illustrates an anterior-anterior band 40b having sections 42a and 42b, each section has a prismatic configuration designed to be applied to and/or extended over anterior of the patient. In practice, sections 42a and 42b may be integrated as a single band, or two attachable bands, such as, for example, along one of the dashed lines as shown via any fastening material (e.g., velcro).

In a third exemplary embodiment, FIG. 2A illustrates an anterior-posterior band 41b having section 43a having a prismatic configuration designed to primarily be applied to and/or extended over an anterior of a patient and a section having a prismatic configuration designed to primarily be applied to and/or extended over a posterior of a patient. In practice, sections 42a and 42b may be integrated as a single band, or two attachable bands, such as, for example, along one of the dashed lines as shown via any fastening material (e.g., velcro).

In practice, any combination of bands 40a, 41a, 40b and 41b may be utilized for defibrillation.

For example, in a fourth exemplary embodiment, FIG. 2A illustrates an integration of anterior-anterior band 40b and anterior-posterior band 41a, whereby anterior-anterior band 40b and anterior-posterior band 41a may be integrated as a single band, or two attachable bands, such as, for example, along one of the dashed lines as shown relative to section 42b and band 41a via any fastening material (e.g., velcro).

Referring to FIG. 2B, four (4) electrode pads 50-53 are shown having a prismatic shape, such as, for example, electrode pads utilized with Automated External Defibrillators (AED) as known in the art of the present disclosure.

In practice, four (4) electrode pads 50-53 will typically be utilized in a defibrillation electrode pad assembly of the present disclosure and therefore will be utilized herein to describe various embodiments of a defibrillation electrode pad assembly of the present disclosure. Nonetheless, those having ordinary skill in the art of the present disclosure will appreciate additional embodiments of defibrillation electrode pad assembly of the present disclosure having three (3) electrode pads and five (5) or more electrode pads.

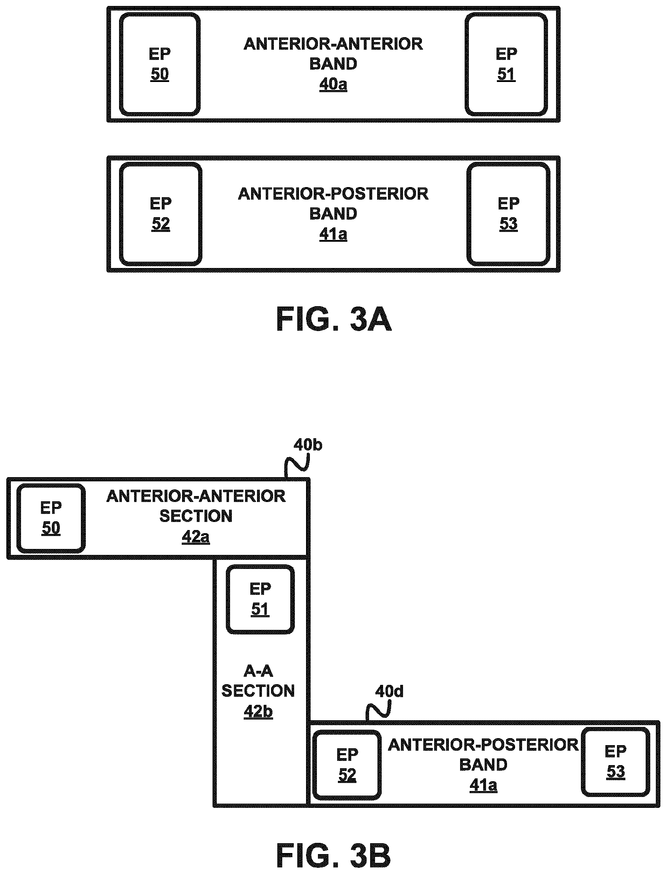

Referring to FIG. 3A, electrode pad 50 and electrode pad 51 of FIG. 2B are shown integrated with anterior-anterior band 40a of FIG. 2A. In practice, electrode pad 50 and electrode pad 51 may be internally disposed within, externally disposed on anterior-anterior band 40a or a combination thereof. Also, while electrode pad 50 and electrode pad 51 are shown in FIG. 3A at the opposite ends of anterior-anterior band 40a, in practice, electrode pad 50 and electrode pad 51 can be disposed at any position within, on or any combination of anterior-anterior band 40a.

Still referring to FIG. 3B, electrode pad 52 and electrode pad 53 of FIG. 2B are shown integrated with anterior-posterior band 40b of FIG. 2A. In practice, electrode pad 52 and electrode pad 53 may be internally disposed within, externally disposed on anterior-posterior band 40b or a combination thereof. Also, while electrode pad 52 and electrode pad 53 are shown in FIG. 3A at the opposite end positions of anterior-posterior band 40b, in practice, electrode pad 52 and electrode pad 53 can be disposed at any position within, on or any combination of anterior-posterior band 40b.

Referring to FIG. 3B, electrode pad 50 of FIG. 2B is shown integrated with section 42a of anterior-anterior pad 40b of FIG. 2A and electrode pad 51 of FIG. 2B is shown integrated with section 42b of anterior-anterior pad 40b of FIG. 2A. In practice, electrode pad 50 and electrode pad 51 may be internally disposed within, externally disposed on anterior-anterior band 40b or a combination thereof. Also, while electrode pad 50 is shown in FIG. 3B at one end position of band section 42a and electrode pad 51 is shown at one end position of band section 42b, in practice, electrode pad 50 and electrode pad 51 can be disposed at any position within, on or any combination of band sections 42a and 42b, respectively.

Still referring to FIG. 3B, electrode pad 52 and electrode pad 53 of FIG. 2B are shown integrated with anterior-posterior band 40b of FIG. 2A. Again, in practice, electrode pad 52 and electrode pad 53 may be internally disposed within, externally disposed on anterior-posterior band 40b or a combination thereof. Also, while electrode pad 52 and electrode pad 53 are shown in FIG. 3A at the opposite end positions of anterior-posterior band 40b, in practice, electrode pad 52 and electrode pad 53 can be disposed at any position within, on or any combination of anterior-posterior band 40b.

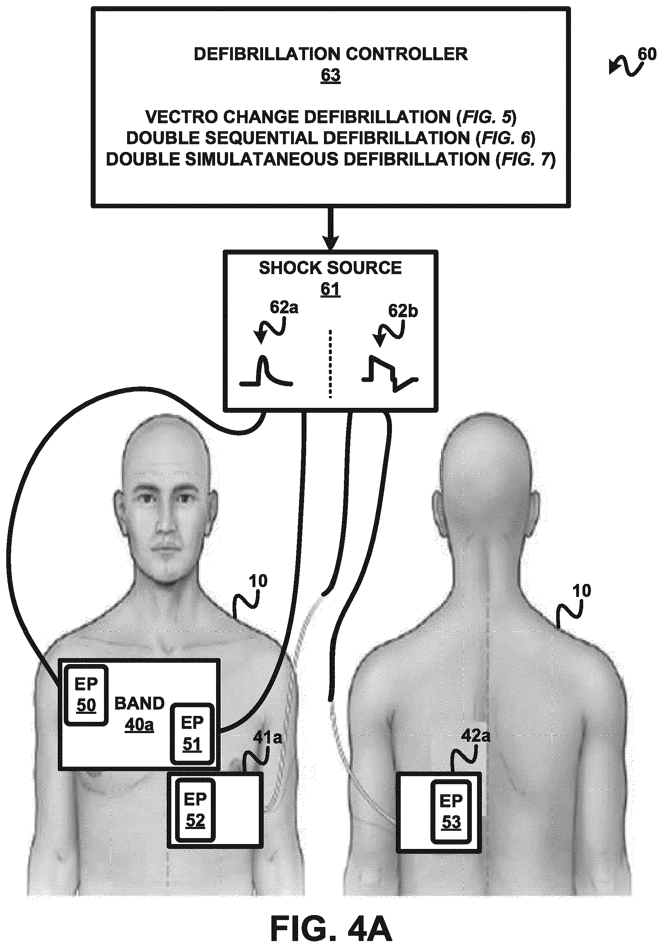

To facilitate a further understanding of the present disclosure, the following description of FIGS. 4A-8 teaches exemplary embodiments of a multiple defibrillation vector therapy system and a multiple defibrillation vector therapy method in accordance with the present disclosure. From the description of FIGS. 4A-8, those having ordinary skill in the art of the present disclosure will appreciate how to apply the present disclosure to make and use additional embodiments of defibrillation electrode pad assembly in accordance with the present disclosure.

Referring to FIG. 4A, a first exemplary multiple defibrillation vector therapy system of the present disclosure in operation employs anterior-anterior band 40a and anterior-posterior band of 41a of FIG. 3A shown placed on a patient 10 and connected to a shock source 61 of defibrillator 60.

Shock source 61 is structurally configured as known in the art of the present disclosure to store electric energy for delivery of a defibrillation shocks via electrode pads 50-53 to a heart of patient 1 as controlled by a defibrillation controller 63 as known in the art of the present disclosure.

In practice, the defibrillation shock may have any waveform as known in the art of the present disclosure. Examples of such waveforms include, but are not limited to, a monophasic sinusoidal waveform (positive sine wave) 62a and a biphasic truncated waveform 62b as shown in FIG. 4A.

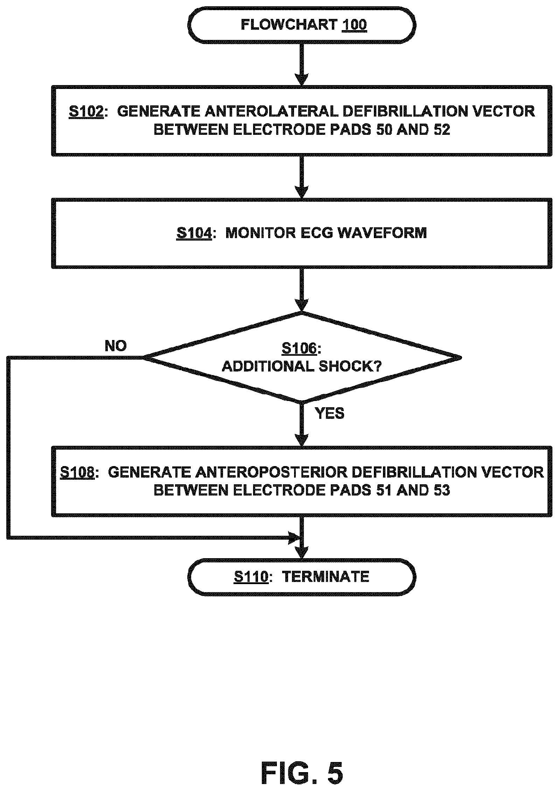

In one exemplary embodiment, shock source 61 employs one or two high voltage capacitor banks (not shown) for storing a high voltage via a high voltage charger and a power supply upon a pressing of a charge button. Shock source 61 further employs a switching/isolation circuit (not shown) for selectively applying a specific waveform of an electric energy charge from a high voltage capacitor bank to either electrode pads 50 and 52 or to electrode pads 51 and 53 as controlled by defibrillation controller 53 as will be exemplary explained with the descriptions of FIG. 5-8 of the present disclosure.

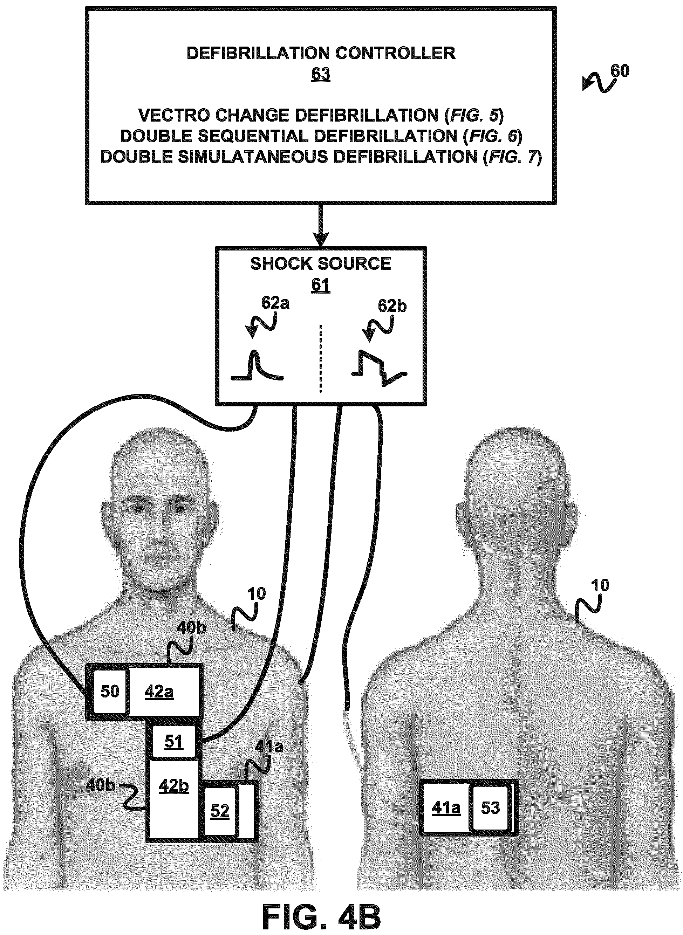

Referring to FIG. 4B, a second exemplary multiple defibrillation vector therapy system of the present disclosure in operation employs anterior-anterior band 40b and anterior-posterior band of 41a of FIG. 3B shown placed on a patient 10 and connected to shock source 61 of defibrillator 60.

As with FIG. 3A, shock source 61 is structurally configured as known in the art of the present disclosure to store electric energy for delivery of a defibrillation shock via electrode pads 50-53 to a heart of patient 1 as controlled by a defibrillation controller 63 as known in the art of the present disclosure.

As with FIG. 3A, in one exemplary embodiment, shock source 61 employs one or two high voltage capacitor banks (not shown) for storing a high voltage via a high voltage charger and a power supply upon a pressing of a charge button. Shock source 61 further employs a switching/isolation circuit (not shown) for selectively applying a specific waveform of an electric energy charge from a high voltage capacitor bank to either electrode pads 50 and 52 or to electrode pads 51 and 53 as controlled by defibrillation controller 53 as will be exemplary explained with the descriptions of FIG. 5-8 of the present disclosure.

In practice, defibrillation controller 63 may implement one or more manual or an automated single shock delivery(ies) to either electrode pads 50 and 52 or electrode pads 51 and 53 as known in the art of the present disclosure.

Also in practice, defibrillation controller 63 may implement one or more manual or an automated double shock delivery(ies) to either electrode pads 50 and 52 or electrode pads 51 and 53 in execution of a vector-change defibrillation, a double sequential defibrillation or a double simultaneous defibrillation of the present disclosure.

FIG. 5 is a flowchart 100 representative of an exemplary use of a vector-change defibrillation system of the present disclosure.

Referring to FIGS. 4A, 4B and 5, during a cardiac treatment of patient 10, flowchart 100 is started upon an initial decision by defibrillation controller 63 that a shock delivery to patient 10 is warranted based on one or more techniques as known in the art of the present disclosure or upon a manual control of defibrillator 60 for a shock delivery to patient 10 as known in the art of the present disclosure.

A stage S102 of flowchart 100 encompasses defibrillation controller 63 controlling a generation of an anterolateral defibrillation vector between electrode pads 50 and 52 as known in the art of the present disclosure followed by a stage S104 of flowchart 100 encompassing defibrillation controller 63 monitoring an ECG waveform of the patient during a cardiac treatment of the patient as known in the art of the present disclosure, particularly during a CPR of the patient.

If defibrillation controller 63 determines, as known in the art of the present disclosure during a stage S106, that an additional shock is not warranted prior to a conclusion of the cardiac treatment of the patient, then defibrillation controller 63 proceeds to a stage S110 of flowchart 100 to terminate flowchart 100.

Otherwise, if defibrillation controller 63 determines, as known in the art of the present disclosure during stage S106, that an additional shock is warranted prior to a conclusion of the cardiac treatment of the patient, then defibrillation controller 63 proceeds to a stage S108 of flowchart 100 control a generation of an anteroposterior defibrillation vector between electrode pads 51 and 53 as known in the art of the present disclosure and then terminates flowchart 100.

Defibrillation controller 63 may restart flowchart 100 if any additional shock(s) is(are) warranted upon to a conclusion of the cardiac treatment of the patient.

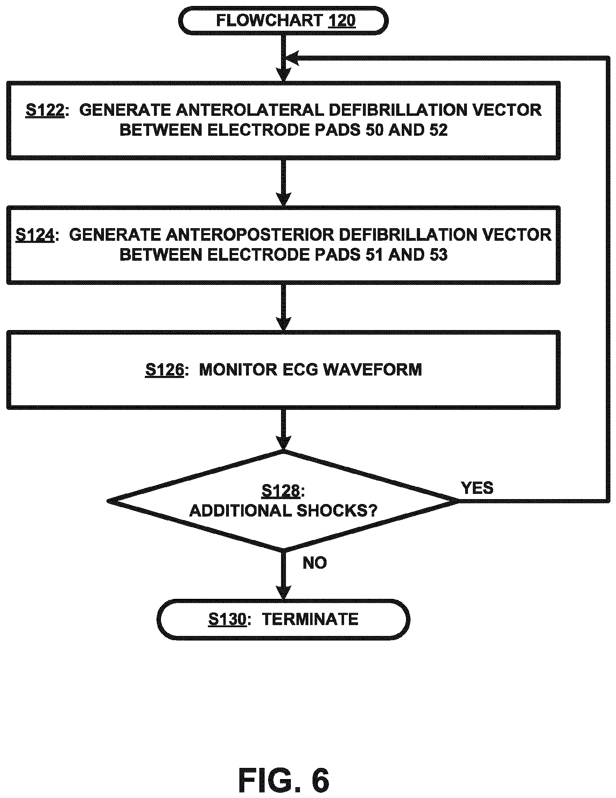

FIG. 6 is a flowchart 120 representative of a first exemplary use of a double sequential defibrillation system of the present disclosure.

Referring to FIGS. 4A, 4B and 6, during a cardiac treatment of patient 12, flowchart 120 is started upon an initial decision by defibrillation controller 63 that a double sequential defibrillation to patient 12 is warranted based on one or more techniques as known in the art of the present disclosure or upon a manual control of defibrillator 60 for double sequential defibrillation to patient 12 as known in the art of the present disclosure.

A stage S122 of flowchart 120 encompasses defibrillation controller 63 controlling a generation of an anterolateral defibrillation vector between electrode pads 50 and 52 as known in the art of the present disclosure immediately followed by defibrillation controller 63 controlling a generation of an anteroposterior defibrillation vector of between electrode pads 51 and 53 as known in the art of the present disclosure.

Thereafter, a stage S126 of flowchart 120 encompasses defibrillation controller 63 monitoring an ECG waveform of the patient during a cardiac treatment of the patient as known in the art of the present disclosure, particularly during a CPR of the patient.

If defibrillation controller 63 determines, as known in the art of the present disclosure during a stage S128, that additional shocks are not warranted prior to a conclusion of the cardiac treatment of the patient, then defibrillation controller 63 proceeds to a stage S130 of flowchart 120 to terminate flowchart 120.

Otherwise, if defibrillation controller 63 determines, as known in the art of the present disclosure during stage S128, that an additional shock is warranted prior to a conclusion of the cardiac treatment of the patient, then defibrillation controller 63 returns to stages S122-S126 to deliver the double sequential defibrillation shocks and monitor the ECG waveform.

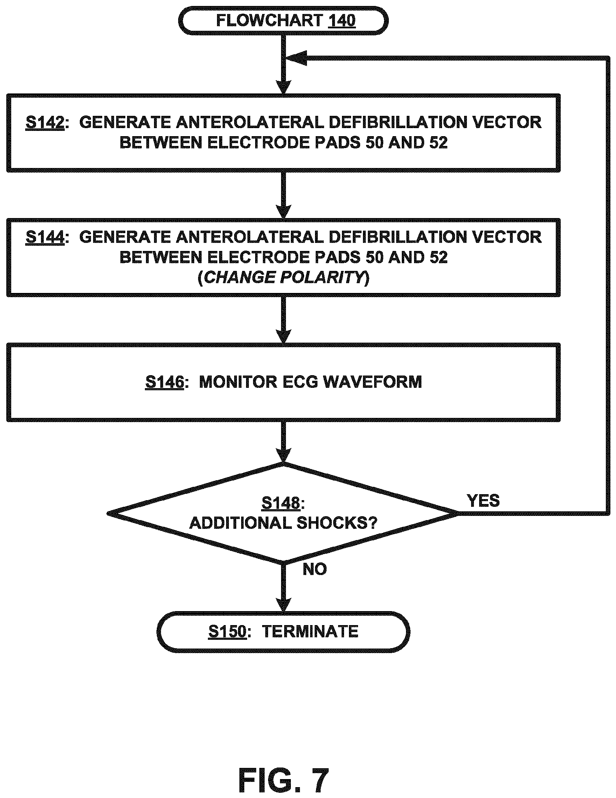

FIG. 7 is a flowchart 140 representative of a second exemplary use of a double sequential defibrillation system of the present disclosure.

Referring to FIGS. 4A, 4B and 7, during a cardiac treatment of patient 14, flowchart 140 is started upon an initial decision by defibrillation controller 63 that a double sequential defibrillation to patient 14 is warranted based on one or more techniques as known in the art of the present disclosure or upon a manual control of defibrillator 60 for double sequential defibrillation to patient 14 as known in the art of the present disclosure.

A stage S142 of flowchart 140 encompasses defibrillation controller 63 controlling a generation of a first anterolateral defibrillation vector between electrode pads 50 and 52 as known in the art of the present disclosure immediately followed by a stage S144 of flowchart 140 encompassing defibrillation controller 63 controlling a generation of a second anterolateral defibrillation vector of an opposite polarity between electrode pads 50 and 52 as known in the art of the present disclosure.

Thereafter, a stage S146 of flowchart 140 encompasses defibrillation controller 63 monitoring an ECG waveform of the patient during a cardiac treatment of the patient as known in the art of the present disclosure, particularly during a CPR of the patient.

If defibrillation controller 63 determines, as known in the art of the present disclosure during a stage S148, that additional shocks are not warranted prior to a conclusion of the cardiac treatment of the patient, then defibrillation controller 63 proceeds to a stage S150 of flowchart 140 to terminate flowchart 140.

Otherwise, if defibrillation controller 63 determines, as known in the art of the present disclosure during stage S148, that an additional shock is warranted prior to a conclusion of the cardiac treatment of the patient, then defibrillation controller 63 returns to stages S142-S146 to deliver the double sequential defibrillation shocks and to monitor the ECG waveforms.

In practice, those having ordinary skill in the art of the present disclosure will appreciate flowchart 140 is applicable to electrode pads 51 and 53.

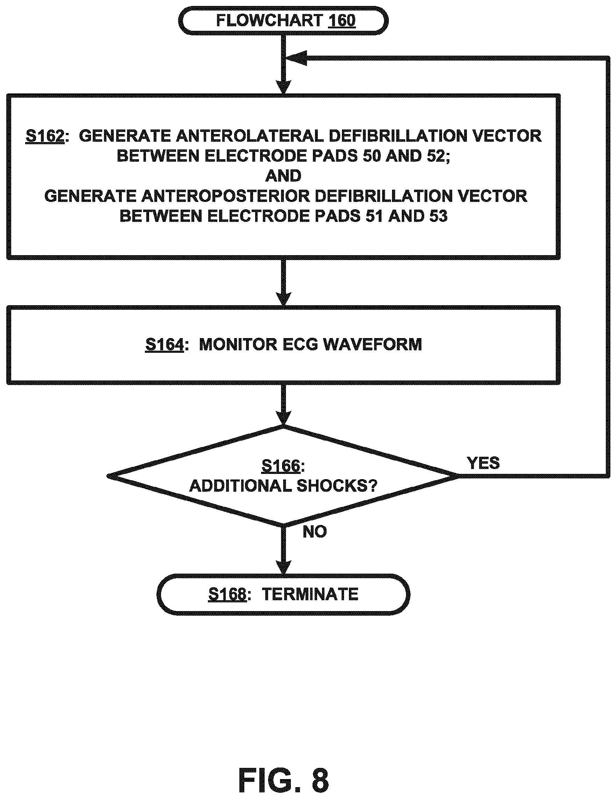

FIG. 8 is a flowchart 160 representative of an exemplary use of a double simultaneous defibrillation system of the present disclosure.

Referring to FIGS. 4A, 4B and 8, during a cardiac treatment of patient 16, flowchart 160 is started upon an initial decision by defibrillation controller 63 that a double simultaneous defibrillation to patient 16 is warranted based on one or more techniques as known in the art of the present disclosure or upon a manual control of defibrillator 60 for double simultaneous defibrillation to patient 16 as known in the art of the present disclosure.

A stage S162 of flowchart 160 encompasses defibrillation controller 63 controlling a simultaneous generation of an anterolateral defibrillation vector between electrode pads 50 and 52 as known in the art of the present disclosure and a generation of an anteroposterior defibrillation vector of between electrode pads 51 and 53 as known in the art of the present disclosure.

Thereafter, a stage S164 of flowchart 160 encompasses defibrillation controller 63 monitoring an ECG waveform of the patient during a cardiac treatment of the patient as known in the art of the present disclosure, particularly during a CPR of the patient.

If defibrillation controller 63 determines, as known in the art of the present disclosure during a stage S165, that additional shocks are not warranted prior to a conclusion of the cardiac treatment of the patient, then defibrillation controller 63 proceeds to a stage S 168 of flowchart 160 to terminate flowchart 160.

Otherwise, if defibrillation controller 63 determines, as known in the art of the present disclosure during stage S166, that an additional shock is warranted prior to a conclusion of the cardiac treatment of the patient, then defibrillation controller 63 returns to stages S162 and S 164 to deliver the double simultaneous defibrillation shocks and monitor the ECG waveform.

From the description of FIGS. 2A-8 herein, those having ordinary skill in the art will appreciate the numerous benefits of the present disclosure including, but not limited to, a defibrillation electrode pad therapy that facilitates multiple defibrillation vector therapy including a vector-change defibrillation (excluding any movement of electrodes on a patient), a double sequential defibrillation (particularly, from a single defibrillator) and/or a double simultaneous defibrillation (particularly, from a single defibrillator).

The present disclosure has been described with reference to the preferred embodiments. Modifications and alterations may occur to others upon reading and understanding the preceding detailed description. It is intended that the invention be construed as including all such modifications and alterations insofar as they come within the scope of the appended claims or the equivalents thereof.

Further, as one having ordinary skill in the art shall appreciate in view of the teachings provided herein, features, elements, components, etc. disclosed and described in the present disclosure/specification and/or depicted in the appended Figures and/or recited in the Claims can be implemented in various combinations of hardware and software, and provide functions which may be combined in a single element or multiple elements. For example, the functions of the various features, elements, components, etc. shown/illustrated/depicted in the Figures and/or recited in the Claims can be provided through the use of dedicated hardware as well as hardware capable of executing software in association with appropriate software. When provided by a processor, the functions can be provided by a single dedicated processor, by a single shared processor, or by a plurality of individual processors, some of which can be shared and/or multiplexed. Moreover, explicit use of the term "processor" or "controller" should not be construed to refer exclusively to hardware capable of executing software, and can implicitly include, without limitation, digital signal processor ("DSP") hardware, memory (e.g., read only memory ("ROM") for storing software, random access memory ("RAM"), non-volatile storage, etc.) and virtually any means and/or machine (including hardware, software, firmware, combinations thereof, etc.) which is capable of (and/or configurable) to perform and/or control a process.

Moreover, all statements herein reciting principles, aspects, and exemplary embodiments of the present disclosure, as well as specific examples thereof, are intended to encompass both structural and functional equivalents thereof. Additionally, it is intended that such equivalents include both currently known equivalents as well as equivalents developed in the future (e.g., any elements developed that can perform the same or substantially similar functionality, regardless of structure). Thus, for example, it will be appreciated by one having ordinary skill in the art in view of the teachings provided herein that any block diagrams presented herein can represent conceptual views of illustrative system components and/or circuitry embodying the principles of the invention. Similarly, one having ordinary skill in the art should appreciate in view of the teachings provided herein that any flow charts, flow diagrams and the like can represent various processes which can be substantially represented in computer readable storage media and so executed by a computer, processor or other device with processing capabilities, whether or not such computer or processor is explicitly shown.

Having described preferred and exemplary embodiments of the present disclosure, which embodiments are intended to be illustrative and not limiting, it is noted that modifications and variations can be made by persons having ordinary skill in the art in view of the teachings provided herein, including the appended Figures and claims. It is therefore to be understood that changes can be made in/to the preferred and exemplary embodiments of the present disclosure which are within the scope of the present disclosure and exemplary embodiments disclosed, described and taught herein.

Moreover, it is contemplated that corresponding and/or related systems incorporating and/or implementing the device or such as may be used/implemented in a device in accordance with the present disclosure are also contemplated and considered to be within the scope of the present disclosure.

## Claims

1. A defibrillation electrode pad assembly for a multiple defibrillation vector therapy for a patient, the defibrillation electrode pad assembly comprising:
an anterior-anterior band (40);
a first electrode pad (50) integrated with the anterior-anterior band (40) and configured to be placeable on an anterior of the patient;
a second electrode pad (51) integrated with the anterior-anterior band (40) and configured to be placeable on the anterior of the patient;
an anterior-posterior band (41);
a third electrode pad (52) integrated with the anterior-posterior band (41) and configured to be placeable on the anterior of the patient;
a fourth electrode pad (53) integrated with the anterior-posterior band (41) and configured to be placeable on a posterior of the patient;
wherein the first electrode pad (50) and the third electrode pad (52) are operable to generate an anterior-anterior defibrillation vector between the first electrode pad (50) and the third electrode pad (52) when the anterior-anterior band (40) and the anterior-posterior band (41) are utilized to place the first electrode pad (50) and the third electrode pad (52) on the anterior of the patient; and
wherein the second electrode pad (51) and the fourth electrode pad (53) are operable to generate an anterior-posterior defibrillation vector between the second electrode pad (51) and the fourth electrode pad (53) when the anterior-anterior band (40) and the anterior-posterior band (41) are utilized to place the second electrode pad (51) on the anterior of the patient and the fourth electrode pad (53) on the posterior of the patient.

2. The defibrillation electrode pad assembly of claim 1, wherein the anterior-anterior band (40) and the anterior-posterior band (41) are segregated.

3. The defibrillation electrode pad assembly of claim 1, wherein the anterior-anterior band (40) and the anterior-posterior band (41) are integrated.

4. The defibrillation electrode pad assembly of claim 3,
wherein the anterior-anterior band (40) further includes a horizontal section and a vertical section oblique to the horizontal section;
wherein the first electrode pad (50) is integrated with the horizontal section; and
wherein the second electrode pad (51) is integrated with the vertical section.

5. The defibrillation electrode pad assembly of claim 1, wherein at least one of the anterior-anterior band (40) and the anterior-posterior band (41) is flexible.

6. The defibrillation electrode pad assembly of claim 1, wherein at least one of:
the first electrode pad (50) is removably attached to the anterior-anterior band (40);
the second electrode pad (51) is removably attached to anterior-anterior band (40);
the third electrode pad (52) is removably attached to the anterior-posterior band (41); and
the fourth electrode pad (53) is removably attached to the anterior-posterior band (41).

7. A multiple defibrillation vector therapy system of a patient, the multiple defibrillation vector therapy system comprising:
a defibrillation electrode pad assembly as claimed in any of the preceding claims; and
a defibrillator (60),
wherein the defibrillator (60) is configured to control a generation of an anterior-anterior defibrillation vector between the first electrode pad (50) and the third electrode pad (52) when the anterior-anterior band (40) and the anterior-posterior band (41) are utilized to place the first electrode pad (50) and the third electrode pad (52) on the anterior of the patient; and
wherein the defibrillator (60) is further configured to control a generation of an anterior-posterior defibrillation vector between the second electrode pad (51) and the fourth electrode pad (53) when the anterior-anterior band (40) and the anterior-posterior band (41) are utilized to place the second electrode pad (51) on the anterior of the patient and the fourth electrode pad (53) on the posterior of the patient.

8. The multiple defibrillation vector therapy system of claim 7, wherein the defibrillator (60) is configured to execute a vector change defibrillation of the patient based on the anterior-anterior defibrillation vector and the anterior-posterior defibrillation vector.

9. The multiple defibrillation vector therapy system of claim 7, wherein the defibrillator (60) is configured to execute a double sequential defibrillation of the patient based on at least one of the anterior-anterior defibrillation vector and the anterior-posterior defibrillation vector.

10. The multiple defibrillation vector therapy system of claim 7, wherein the defibrillator (60) is configured to execute a double simultaneous defibrillation of the patient based on at least one of the anterior-anterior defibrillation vector and the anterior-posterior defibrillation vector.

## Patentansprüche

1. Ein Defibrillations-Elektrodenpolsteraufbau für eine Therapie mit mehreren Defibrillationsvektoren für einen Patienten, wobei der Defibrillations-Elektrodenpolsteraufbau Folgendes umfasst:
ein Vorderseiten-Vorderseiten-Band (40);
ein erstes Elektrodenpolster (50), integriert in das Vorderseiten-Vorderseiten-Band (40) und ausgebildet, um auf eine Vorderseite des Patienten positionierbar zu sein;
ein zweites Elektrodenpolster (51), integriert in das Vorderseiten-Vorderseiten-Band (40) und ausgebildet, um auf die Vorderseite des Patienten positionierbar zu sein;
ein Vorderseiten-Rückseiten-Band (41);
ein drittes Elektrodenpolster (52), integriert in das Vorderseiten-Rückseiten-Band (41) und ausgebildet, um auf die Vorderseite des Patienten positionierbar zu sein;
ein viertes Elektrodenpolster (53), integriert in das Vorderseiten-Rückseiten-Band (41) und ausgebildet, um auf eine Rückseite des Patienten positionierbar zu sein;
wobei das erste Elektrodenpolster (50) und das dritte Elektrodenpolster (52) arbeiten, um einen Vorderseiten-Vorderseiten-Defibrillationsvektor zwischen dem ersten Elektrodenpolster (50) und dem dritten Elektrodenpolster (52) zu erzeugen, wenn das Vorderseiten-Vorderseiten-Band (40) und das Vorderseiten-Rückseiten-Band (41) genutzt werden, um das erste Elektrodenpolster (50) und das dritte Elektrodenpolster (52) auf die Vorderseite des Patienten zu platzieren; und
wobei das zweite Elektrodenpolster (51) und das vierte Elektrodenpolster (53) arbeiten, um einen Vorderseiten-Rückseiten-Defibrillationsvektor zwischen dem zweiten Elektrodenpolster (51) und dem vierten Elektrodenpolster (53) zu erzeugen, wenn das Vorderseiten-Vorderseiten-Band (40) und das Vorderseiten-Rückseiten-Band (41) genutzt werden, um das zweite Elektrodenpolster (51) auf die Vorderseite des Patienten und das vierte Elektrodenpolster (53) auf die Rückseite des Patienten zu platzieren.

2. Der Defibrillations-Elektrodenpolsteraufbau gemäß Anspruch 1, wobei das Vorderseiten-Vorderseiten-Band (40) und das Vorderseiten-Rückseiten-Band (41) getrennt sind.

3. Der Defibrillations-Elektrodenpolsteraufbau gemäß Anspruch 1, wobei das Vorderseiten-Vorderseiten-Band (40) und das Vorderseiten-Rückseiten-Band (41) integriert sind.

4. Der Defibrillations-Elektrodenpolsteraufbau gemäß Anspruch 3,
wobei das Vorderseiten-Vorderseiten-Band (40) weiter einen horizontalen Abschnitt und einen vertikalen Abschnitt, schräg zu dem horizontalen Abschnitt, einschließt,
wobei das erste Elektrodenpolster (50) in den horizontalen Abschnitt integriert ist und
wobei das zweite Elektrodenpolster (51) in den vertikalen Abschnitt integriert ist.

5. Der Defibrillations-Elektrodenpolsteraufbau gemäß Anspruch 1, wobei das Vorderseiten-Vorderseiten-Band (40) und/oder das Vorderseiten-Rückseiten-Band (41) elastisch sind.

6. Der Defibrillations-Elektrodenpolsteraufbau gemäß Anspruch 1, wobei mindestens eines von Folgendem gilt:
das erste Elektrodenpolster (50) ist lösbar am Vorderseiten-Vorderseiten-Band (40) befestigt,
das zweite Elektrodenpolster (51) ist lösbar am Vorderseiten-Vorderseiten-Band (40) befestigt,
das dritte Elektrodenpolster (52) ist lösbar am Vorderseiten-Rückseiten-Band (41) befestigt, und
das vierte Elektrodenpolster (53) ist lösbar am Vorderseiten-Rückseiten-Band (41) befestigt.

7. Ein Therapiesystem für einen Patienten, mit mehreren Defibrillationsvektoren, wobei das Therapiesystem mit mehreren Defibrillationsvektoren Folgendes umfasst:
einen Defibrillations-Elektrodenpolsteraufbau gemäß einem beliebigen der obigen Ansprüche und
einen Defibrillator (60),
wobei der Defibrillator (60) konfiguriert ist, um eine Erzeugung eines Vorderseiten-Vorderseiten-Defibrillationsvektors zwischen dem ersten Elektrodenpolster (50) und dem dritten Elektrodenpolster (52) zu steuern, wenn das Vorderseiten-Vorderseiten-Band (40) und das Vorderseiten-Rückseiten-Band (41) genutzt werden, um das erste Elektrodenpolster (50) und das dritte Elektrodenpolster (52) auf die Vorderseite des Patienten zu platzieren; und
wobei der Defibrillator (60) weiter konfiguriert ist, um eine Erzeugung eines Vorderseiten-Rückseiten-Defibrillationsvektors zwischen dem zweiten Elektrodenpolster (51) und dem vierten Elektrodenpolster (53) zu steuern, wenn das Vorderseiten-Vorderseiten-Band (40) und das Vorderseiten-Rückseiten-Band (41) genutzt werden, um das zweite Elektrodenpolster (51) auf die Vorderseite des Patienten und das vierte Elektrodenpolster (53) auf die Rückseite des Patienten zu platzieren.

8. Das Therapiesystem mit mehreren Defibrillationsvektoren gemäß Anspruch 7, wobei der Defibrillator (60) konfiguriert ist, um eine Vektoränderungs-Defibrillation beim Patienten auf der Grundlage des Vorderseiten-Vorderseiten-Defibrillationsvektors und des Vorderseiten-Rückseiten-Defibrillationsvektors vorzunehmen.

9. Das Therapiesystem mit mehreren Defibrillationsvektoren gemäß Anspruch 7, wobei der Defibrillator (60) konfiguriert ist, um eine doppelte sequentielle Defibrillation beim Patienten anhand des Vorderseiten-Vorderseiten-Defibrillationsvektors und/oder des Vorderseiten-Rückseiten-Defibrillationsvektors vorzunehmen.

10. Das Therapiesystem mit mehreren Defibrillationsvektoren gemäß Anspruch 7, wobei der Defibrillator (60) konfiguriert ist, um eine doppelte gleichzeitige Defibrillation beim Patienten anhand des Vorderseiten-Vorderseiten-Defibrillationsvektors und/oder des Vorderseiten-Rückseiten-Defibrillationsvektors vorzunehmen.

## Revendications

1. Assemblage des patchs d'électrodes de défibrillation destiné à une thérapie par vecteurs de défibrillation multiples chez un patient, ledit assemblage des patchs d'électrodes de défibrillation comprenant :
une bande antéro-antérieure (40) ;
un premier patch d'électrode (50) intégré à la bande antéro-antérieure (40) et configuré pour être placée sur la partie antérieure du patient ;
un deuxième patch d'électrode (51) intégré à la bande antéro-antérieure (40) et conçu pour être placé sur la partie antérieure du patient ;
une bande antéro-postérieure (41) ;
un troisième patch d'électrode (52) intégré à la bande antéro-postérieure (41) et conçu pour être placé sur la partie antérieure du patient ;
un quatrième patch d'électrode (53) intégré à la bande antéro-postérieure (41) et conçu pour être placé sur la partie postérieure du patient ;
où le premier patch d'électrode (50) et le troisième patch d'électrode (52) sont aptes à générer un vecteur de défibrillation antéro-antérieur entre le premier patch d'électrode (50) et le troisième patch d'électrode (52) lorsque la bande antéro-antérieure (40) et la bande antéro-postérieure (41) sont utilisées pour positionner le premier patch d'électrode (50) et le troisième patch d'électrode (52) sur la partie antérieure du patient ; et
où le deuxième patch d'électrode (51) et le quatrième patch d'électrode (53) sont aptes à générer un vecteur de défibrillation antéro-postérieur entre le deuxième patch d'électrode (51) et le quatrième patch d'électrode (53) lorsque la bande antéro-antérieure (40) et la bande antéro-postérieure (41) sont utilisées pour placer le deuxième patch d'électrode (51) sur la partie antérieure du patient et le quatrième patch d'électrode (53) sur la partie postérieure du patient.

2. Assemblage des patchs d'électrodes de défibrillation selon la revendication 1, où la bande antéro-antérieure (40) et la bande antéro-postérieure (41) sont séparées.

3. Assemblage des patchs d'électrodes de défibrillation selon la revendication 1, où la bande antéro-antérieure (40) et la bande antéro-postérieure (41) sont intégrées.

4. Assemblage des patchs d'électrodes de défibrillation selon la revendication 3,
où la bande antéro-antérieure (40) comprend en outre une section horizontale et une section verticale oblique par rapport à la section horizontale ;
où le premier patch d'électrode (50) est intégré à la section horizontale ; et
où le deuxième patch d'électrode (51) est intégré à la section verticale.

5. Assemblage des patchs d'électrodes de défibrillation selon la revendication 1, où au moins l'une parmi la bande antéro-antérieure (40) et la bande antéro-postérieure (41) est flexible.

6. Assemblage des patchs d'électrodes de défibrillation selon la revendication 1, où au moins l'un des éléments suivants :
le premier patch d'électrode (50) est fixé de manière amovible à la bande antéro-antérieure (40) ;
le deuxième patch d'électrode (51) est fixé de manière amovible à la bande antéro-antérieure (40) ;
le troisième patch d'électrode (52) est fixé de manière amovible à la bande antéro-postérieure (41) ; et
le quatrième patch d'électrode (53) est fixé de manière amovible à la bande antéro-postérieure (41).

7. Système de thérapie par vecteurs de défibrillation multiples destiné à un patient, le système de thérapie par vecteurs de défibrillation multiples comprenant :
un assemblage des patchs d'électrodes de défibrillation selon l'une quelconque des revendications précédentes ; et
un défibrillateur (60),
où le défibrillateur (60) est configuré pour commander la génération d'un vecteur de défibrillation antéro-antérieur entre le premier patch d'électrode (50) et le troisième patch d'électrode (52) lorsque la bande antéro-antérieure (40) et la bande antéro-postérieure (41) sont utilisées pour positionner le premier patch d'électrode (50) et le troisième patch d'électrode (52) sur la partie antérieure du patient ; et
où le défibrillateur (60) est en outre configuré pour commander la génération d'un vecteur de défibrillation antéro-postérieur entre le deuxième patch d'électrode (51) et le quatrième patch d'électrode (53) lorsque la bande antéro-antérieure (40) et la bande antéro-postérieure (41) sont utilisées pour placer le deuxième patch d'électrode (51) sur la partie antérieure du patient et le quatrième patch d'électrode (53) sur la partie postérieure du patient.

8. Système de thérapie par vecteurs de défibrillation multiples selon la revendication 7, où le défibrillateur (60) est configuré pour exécuter une défibrillation avec changement de vecteur sur le patient sur la base du vecteur de défibrillation antéro-antérieur et du vecteur de défibrillation antéro-postérieur.

9. Système de thérapie par vecteurs de défibrillation multiples selon la revendication 7, où le défibrillateur (60) est configuré pour effectuer une double défibrillation séquentielle du patient sur la base d'au moins l'un parmi le vecteur de défibrillation antéro-antérieur et le vecteur de défibrillation antéro-postérieur.

10. Système de thérapie par vecteurs de défibrillation multiples selon la revendication 7, où le défibrillateur (60) est configuré pour effectuer une double défibrillation simultanée du patient sur la base d'au moins l'un parmi le vecteur de défibrillation antéro-antérieur et le vecteur de défibrillation antéro-postérieur.
